# EUROPEAN PATENT SPECIFICATION

(11) **EP 1 779 813 B1**
(45) Date of publication and mention of the grant of the patent: **03.12.2008**
(21) Application number: 06255177.5
(22) Date of filing: 06.10.2006
(51) Int. Cl.: A61F 2/38

(54) **Orthopaedic implant systems with anti-abrasion studs**
Othopädisches Implantiersystem mit Verschleissschutzbolzen
Système d'implant orthopédique avec des goujons anti-abrasifs

(30) Priority: 27.10.2005 US 260386
(43) Date of publication of application: 02.05.2007
(73) Proprietor: DePuy Products, Inc., Warsaw, IN 46581 (US)
(72) Inventor: Rhodes, James M., Warsaw, IN 46580 (US)
(74) Representative: Belcher, Simon James

(56) References cited:
- DE-A1- 2 933 174
- GB-A- 1 522 497
- US-A- 5 108 441
- US-A1- 2005 177 242

## Description

This invention relates generally to prostheses for human body joints, and more particularly, to prostheses for human knees.

When a human skeletal joint is damaged, whether as a result of an accident or illness, a prosthetic replacement of the damaged joint may be necessary to relieve pain and to restore normal use to the joint. Typically the entire joint is replaced by means of a surgical procedure that involves removal of the ends of the corresponding damaged bones and replacement of these ends with prosthetic implants. This replacement of a native joint with a prosthetic joint is referred to as a primary total-joint arthroplasty.

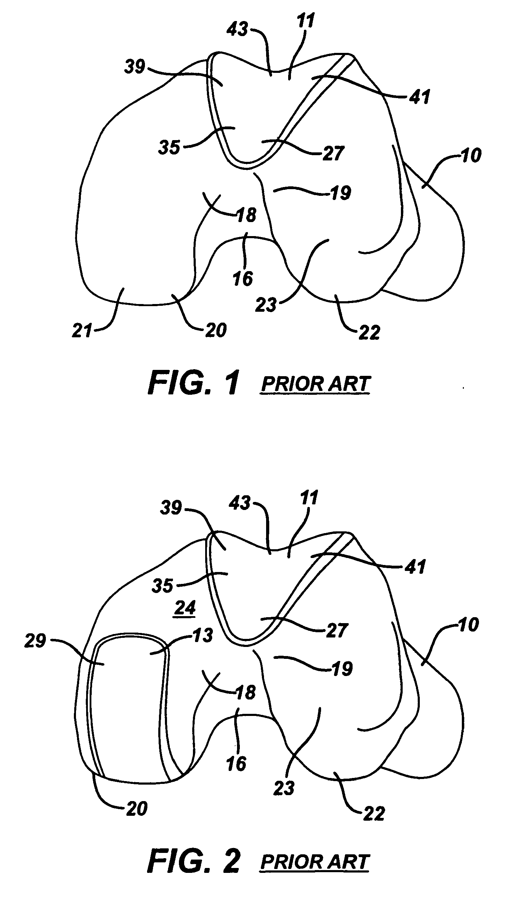

For a damaged human knee, the total knee is commonly replaced with prosthetic components shaped to replace portions of the distal femur, proximal tibia and patella. Prosthetic components for use in replacing the distal femur are shaped to replace the articulating surfaces (shown at 21, 23 in FIG. 1) of the medial condyle (shown at 20 in FIG. 1), lateral condyle (shown at 22 in FIG. 1) and trochlea, and prosthetic components for use in replacing the proximal tibia are shaped to replace the tibial plateau. Commonly, the tibial component is two piece: one piece is affixed to the bone and the other piece is a bearing with concave surfaces receiving the femoral condyles. Frequently, a portion of the patella is also replaced with a prosthetic component as part of the total knee replacement.

In some patients, only a portion of the knee is damaged or injured. For such patients, individual compartments of the knee may be replaced. For example, the medial or lateral compartment of the knee may be replaced with uni-condylar components that replace the articulating surface of one condyle of the distal femur and one side of the tibial plateau. The patellofemoral compartment may be replaced with a femoral component that replaces a portion of the trochlea and a patellar component that replaces part of the patella. In some instances, two or three unicompartmental components are implanted together in one joint; for example, two sets of uni-condylar components could be implanted together to replace the articulating surfaces of both the medial and lateral sides of the tibio-femoral joint, a trochlear component (and patellar component) and a set of uni-condylar femoral and tibial components could be implanted together, or two sets of uni-condylar components and a trochlear component (and patellar component) could be implanted together. The following journal articles report, among other things: use of patellofemoral components (trochlear component and patellar component) and one or two sets of uni-condylar components, Arciero, Major and Toomey, "Patellofemoral Arthroplasty: A Three-to-Nine Year Follow-Up Study", 236 Clinical Orthopaedics and Related Research, Vol. 236, November 1, 1988, pages 60-71; and two sets of uni-condylar components, Bourne, Rorabeck, Finlay and Nott, "Kinematic I and Oxford Knee Arthroplasty: A 5-8-year Follow-up Study", The Journal of Arthroplasty, Vol. 2, No. 4, December, 1987, pages 285-291, and Shoji, D'Ambrosia and Lipscomb, "Failed Polycentric Total Knee Prostheses", The Journal of Bone and Joint Surgery, Vol. 58-A, No. 6, September 1976, pages 773-777, and Stockley, Douglas and Elson, "Bicondylar St. Georg Sledge Knee Arthroplasty", Clinical Orthopaedics and Related Research, No. 255, June, 1990, pages 228-234.

Other documents which relate to uni-condylar knee implant components or patellofemoral implant components include US-3852830, US-4034418, US-4340978, US-4838891, US-5871541, US-6616696, and US-6709460.

Commercial uni-condylar knee implant components or patellofemoral implant components include those sold under the trade marks LCS UNI, Preservation and LCS PFJ by DePuy Orthopaedics Inc, Patella MOD III and Patella II by Smith & Nephew Richards Inc) and Oxford by Biomet Inc.

When knees are replaced with common total joint prostheses, substantially all of the potential articulating surface of the distal femur is replaced and covered with metal; no native articular cartilage remains exposed in the potential area of articulation. In contrast, when one or more compartments of a knee are replaced with unicompartmental components, substantial areas of native cartilage are not covered by metal, and remain exposed. FIG. illustrates an example of a human femur 10 with an implanted trochlear implant component 11. FIG. 2 illustrates an example of a human femur 10 with an implanted trochlear implant component 11 replacing the articulating surface of the trochlea together with a uni-condylar femoral component 13 replacing the articulating surface of one of the femoral condyles. In FIG. 2, the areas of exposed native tissue include the intercondylar notch 16, and areas 18, 19 of the distal femoral condyles 20, 22 adjacent to the intercondylar notch 16 and an area 24 of the distal femoral condyles 20, 22 lying between the distal portion 27 of the trochlear component 11 and the anterior portion 29 of the uni-condylar femoral component 13. As shown in FIGS. 1-2, the distal portion 27 of the trochlear component 11 generally tapers toward its distal end which is positioned near or within the intercondylar notch 16.

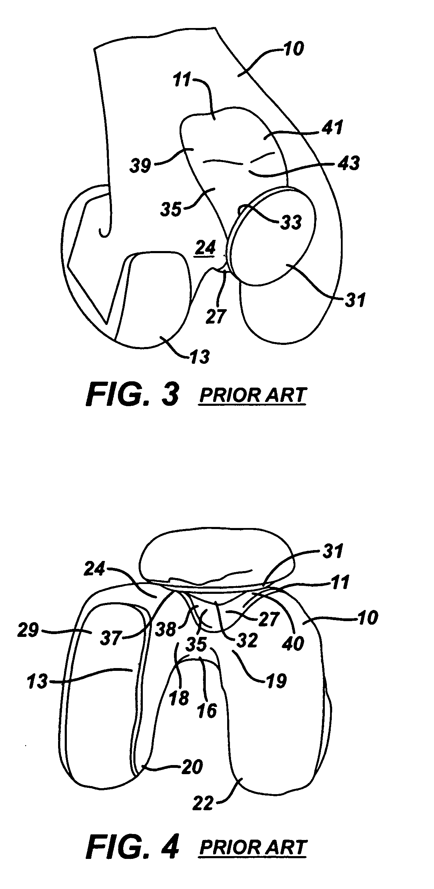

FIG. 3 illustrates the femur 10 of FIG. 2, shown with a patellar implant component 31 engaging the trochlear component 11. The patellar component 31 includes a bearing surface 33 that bears against a bearing surface 35 of the patellar component 11. The exposed bearing surface 35 of the illustrated trochlear implant component 11 has two convex surfaces 39, 41 meeting along a groove 43. FIG. 4 illustrates the femur of FIG. 3 with the patellar component 31 positioned with respect to the trochlear component 11 as it would be with the knee in deep flexion. When the knee is in deep flexion, a portion of the patellar component 31 may extend beyond the edges of the distal portion 27 of the trochlear component 11. Such an overhanging portion (shown at 37 in FIG. 4) of the patellar component 31 may contact and rub against the patient's native tissue (such as native tissue indicated at 18, 19 and 24 in FIG. 4) as the knee flexes and extends. This contact may result in painful irritation of the native tissue. This painful irritation could be prevented through use of a total knee prosthesis; however, use of a total knee prosthesis could result in an unnecessary loss of healthy bone tissue. The pain resulting from this irritation could be treated by revising the surgery, replacing the uni-compartmental components 11, 13 with a total knee prosthesis, again resulting in the loss of healthy bone tissue. A need exists for a means for preventing or treating the patient's native tissue near the intercondylar notch without requiring the removal and replacement of healthy tissue.

US-A-2005/0177242, which is regardes as the closest prior art, discloses a trochlear component with an intercondylar notch portion with tapered wings extending distally and curved posteriorly. The wings also curve away from each other in the posterior direction. Although the wings provide additional bearing surfaces for the patellar implant component, they may not cover the portions of the femur that potentially contact the patellar prosthesis bearing surface. In addition, individual patient anatomies may prevent use of such a trochlear implant in all patients.

The present invention provides an implant system that protects a patient's native tissue when the patient has been treated with uni-compartmental or multi-compartmental arthroplasty. The protection offered by the present invention can be provided in a wide range of patient anatomies.

Accordingly, the invention provides an orthopaedic implant system that includes, in addition to uni-compartmental implant components, one or more anti-abrasion studs that extend the bearing areas of other implant components to protect native tissue from damage resulting from engaging a patellar implant component during flexion and extension. In addition to the anti-abrasion studs, the orthopaedic implant system of the present invention may include a trochlear implant component, a patellar implant component, one or more uni-condylar femoral implant components, and one or more uni-condylar tibial implant components against which the uni-condylar femoral components articulate.

In one aspect, the present invention provides this protection and wide range of use by providing a knee implant system according to claim 1.

Embodiments of the invention will now be described by way of example with reference to the accompanying drawings, in which:
FIG. 1 is a perspective view of a distal femur with an implanted prior art trochlear implant component;
FIG. 2 is a perspective view similar to FIG. 1, showing the distal femur with both a prior art trochlear implant component and a prior art uni-condylar femoral implant component;
FIG. 3 is a perspective view of a distal femur with both a prior art trochlear implant component and a prior art uni-condylar femoral implant component, showing a prior art patellar implant component bearing against the bearing surface of the trochlear implant component;
FIG. 4 is an end view of a distal femur, illustrating a possible position of the patella and prior art patellar implant with respect to a prior art trochlear component and prior art uni-condylar femoral component, and further illustrating the potential for the patellar implant component to contact native tissue during flexion and extension of the knee;
FIG. 5 is a perspective view of a distal femur, similar to FIG. 2, but with a first embodiment of a stud implanted in the space between a trochlear component and uni-condylar implant component;
FIG. 6 is a perspective view of a distal femur, similar to FIGS. 2 and 5, but with two studs of a second embodiment implanted in areas adjacent to the intercondylar notch of the femur;
FIG. 7 is perspective view of a first embodiment of an anti-abrasion stud;
FIG. 8 is an elevation of the anti-abrasion stud of FIG. 7;
FIG. 9 is a second elevation of the anti-abrasion stud of FIGS. 7-8;
FIG. 10 is a cross-section of the anti-abrasion stud of FIG. 9, taken along line 10-10 of FIG. 9;
FIG. 11 is a top plan view of the anti-abrasion stud of FIGS. 7-10;
FIG. 12 is a bottom plan view of the anti-abrasion stud of FIGS. 7-11;
FIG. 13 is perspective view of a second embodiment of an anti-abrasion stud;
FIG. 14 is an elevation of the anti-abrasion stud of FIG. 13;
FIG. 15 is a cross-section of the anti-abrasion stud of FIG. 14, taken along line 15-15 of FIG. 14;
FIG. 16 is a top plan view of the anti-abrasion stud of FIGS. 13-15;
FIG. 17 is a bottom plan view of the anti-abrasion stud of FIGS. 13-16;
FIG. 18 is perspective view of a third embodiment of an anti-abrasion stud;
FIG. 19 is a top plan view of the anti-abrasion stud of FIG. 18;
FIG. 20 is an elevation of the anti-abrasion stud of FIGS. 18-19;
FIG. 21 is a cross-section of the anti-abrasion stud of FIGS. 18-20, taken along line 21-21 of FIG. 20;
FIG. 22 is a bottom plan view of the anti-abrasion stud of FIGS. 18-21;
FIG. 23 is perspective view of a fourth embodiment of an anti-abrasion stud;
FIG. 24 is an elevation of the anti-abrasion stud of FIG. 23;
FIG. 25 is a second elevation of the anti-abrasion stud of FIGS. 23-24;
FIG. 26 is a cross-section of the anti-abrasion stud of FIGS. 23-25, taken along line 26-26 of FIG. 25;
FIG. 27 is a top plan view of the anti-abrasion stud of FIGS. 23-26;
FIG. 28 is a bottom plan view of the anti-abrasion stud of FIGS. 23-27;
FIG. 29 is perspective view of a fifth embodiment of an anti-abrasion stud;
FIG. 30 is an elevation of the anti-abrasion stud of FIG. 29;
FIG. 31 is a second elevation of the anti-abrasion stud of FIGS. 29-30;
FIG. 32 is a top plan view of the anti-abrasion stud of FIGS. 29-31;
FIG. 33 is a bottom plan view of the anti-abrasion stud of FIGS. 29-32; and
FIG. 34 is a cross-section of a portion of a femur and trochlear implant component, illustrating the position of the first embodiment of the anti-abrasion stud with respect to articular cartilage of the femur.

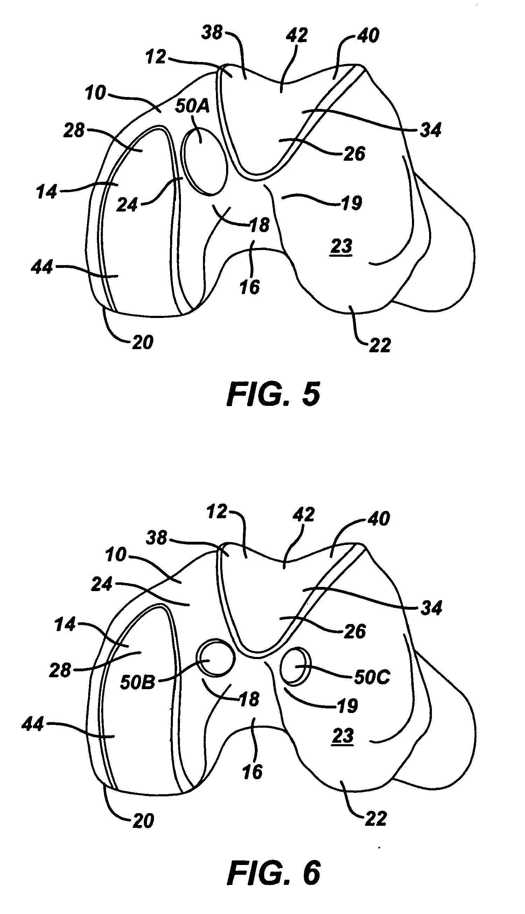

Referring to the drawings, FIG. 5 shows the distal end of a human femur 10, shown with two compartments of the distal femur 10 replaced by a trochlear implant component and a uni-condylar femoral implant component. The illustrated trochlear and uni-condylar implant components of FIG 5. are similar to those disclosed in US-A-2005/ 0154471. However, it should be understood that the present invention is not limited to the structures disclosed in that document; the principles of the present invention, and the addition of anti-abrasion studs 50, can be broadly applied to other implant systems wherein a portion of native tissue is exposed to potential contact with the articulating surface.

The illustrated trochlear implant component 12 is sized and shaped to replace a portion of the patellofemoral compartment of the distal femur without covering the distal articulating surfaces 21, 23 of the medial and lateral condyles 20, 22. The trochlear component 12 has an exposed bearing surface 34 and a bone-facing surface underlying the bearing surface. The exposed bearing surface 34 of the illustrated trochlear implant component 12 has two convex surfaces 38, 40 meeting along a groove 42. The illustrated trochlear implant component 12 is sized and shaped to provide an articulating surface for the patellar component 30, so that the patellar component 30 engages the trochlear component 12 when the leg is in extension as well as through a normal range of flexion.

The illustrated uni-condylar implant component 14 is sized and shaped to replace the femoral condyle surface 21 that articulates with the proximal tibia. The uni-condylar femoral implant component 14 has an exposed arcuate articulating or bearing surface 44 and an underlying bone-facing surface. The bone-facing surface can be porous to promote bone ingrowth, or can be adapted for cemented fixation. Overall, the illustrated uni-condylar femoral implant component 14 is sized and shaped to cover the distal and posterior articulating surfaces of one femoral condyle.

As shown in FIGS. 4-6, the illustrated trochlear component 12 has a distal portion 26 that tapers distally and posteriorly; the illustrated uni-condylar femoral component 14 has an anterior portion 28 that tapers proximally and anteriorly. One end of the illustrated trochlear component 12 is implanted adjacent to the intercondylar notch 16. The intercondylar notch 16 remains in its native state, as does a portion 24 of the femoral condyle between the tapering edges of the trochlear component 12 and the uni-condylar femoral component 14 and as do portions 18, 19 of the distal femur adjacent to the intercondylar notch 16. These portions 18, 19, 24 of the femur in their native state include native tissue, such as articular cartilage.

Although not illustrated in the accompanying drawings, it should be understood that the illustrated uni-condylar femoral implant component 14 would be used in conjunction with a uni-condylar tibial implant component. Such a uni-condylar tibial implant component would typically be two-piece, with a metal base and a polymer bearing made of a material such as ultra-high molecular weight polyethylene (UHMWPE), but could be a single integral implant component made out of a material such as UHMWPE.

When a trochlear component is implanted, the implant system would also typically include a patellar implant component, such as that shown at 30 in FIGS. 3-4. The patellar implant component 30 is sized and shaped to replace a posterior portion of the patella. The patellar implant component has a bearing surface 32 and a bone-facing surface. The illustrated patellar implant component 30 is a two-piece component, with a bearing made out of a smooth material such as (UHMWPE), although the patellar component could be a single integral implant component made out of a material such as UHMWPE.

To protect the area 24 of native tissue between the opposed tapered edges of the distal portion 26 of the trochlear component 12 and anterior portion 28 of the uni-condylar femoral component 14, the orthopaedic implant system of FIG. 5 includes a first embodiment of an anti-abrasion stud 50A implanted at this area 24 of native tissue. To protect the areas 18, 19 of native tissue adjacent the intercondylar notch 16, the orthopaedic implant system of FIG. 6 includes a medial anti-abrasion stud 50B and a lateral anti-abrasion stud 50C implanted at these areas 18, 19. As described in more detail below, other embodiments 50D, 50E of anti-abrasion studs may also be employed to extend the patellar tracking surface and thereby protect native tissue.

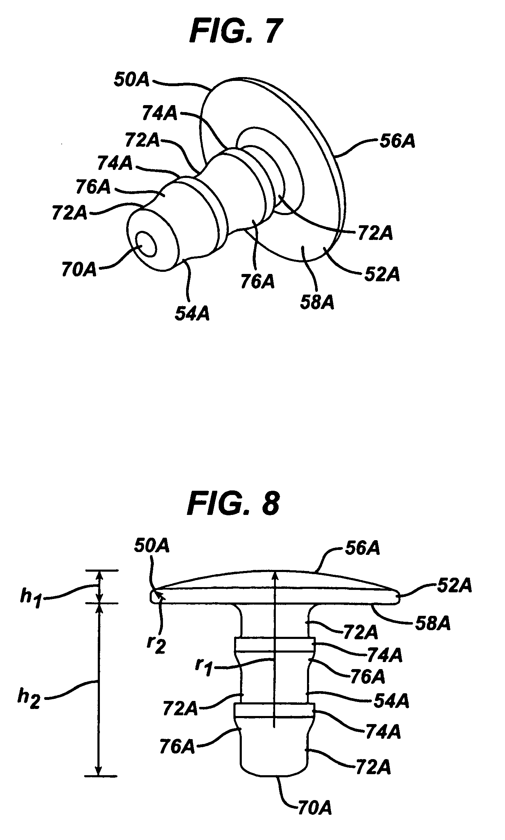

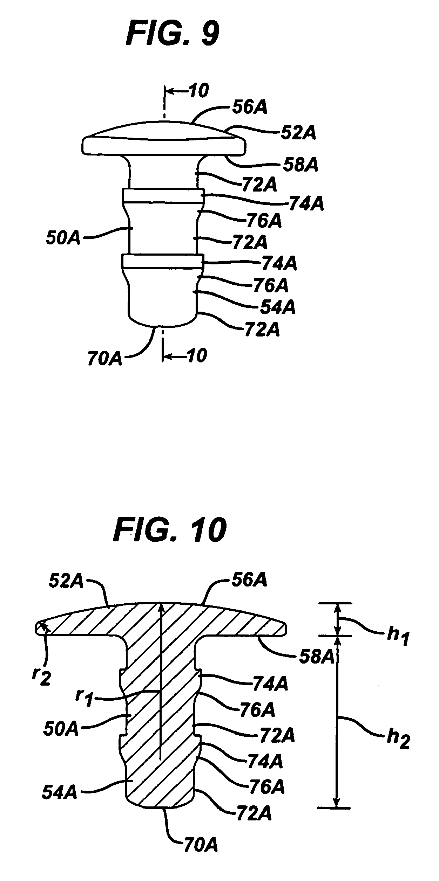

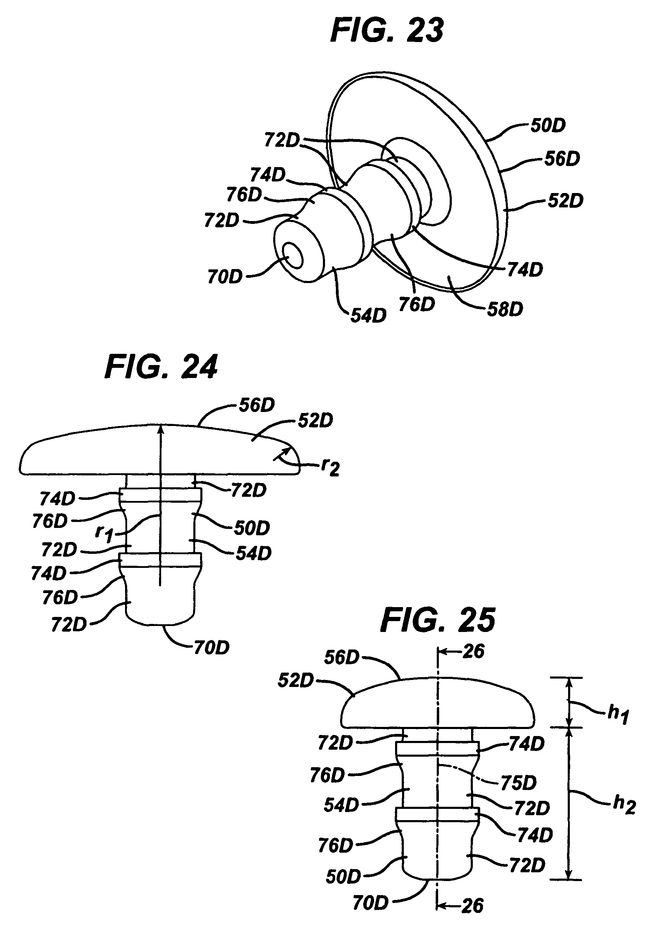

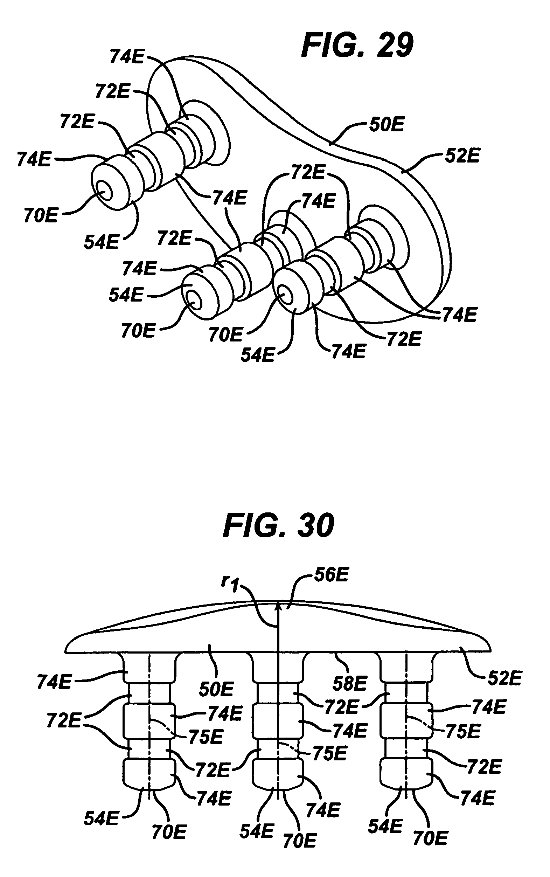

All of the illustrated anti-abrasion studs 50A, 50B, 50C, 50D, 50E include common features. As shown in FIGS. 7-33, they each include a head 52A, 52B, 52C, 52D, 52E and a fixation post 54A, 54B, 54C, 54D, 54E. Each head 52A, 52B, 52C, 52D, 52E has a bearing surface 56A, 56B, 56C, 56D, 56E and an opposite bone-facing surface 58A, 58B, 58C, 58D, 58E. The fixation posts 54A, 54B, 54C, 54D, 54E extend outward from the bone-facing surface 58A, 58B, 58C, 58D, 58E of the head 52A, 52B, 52C, 52D, 52E.

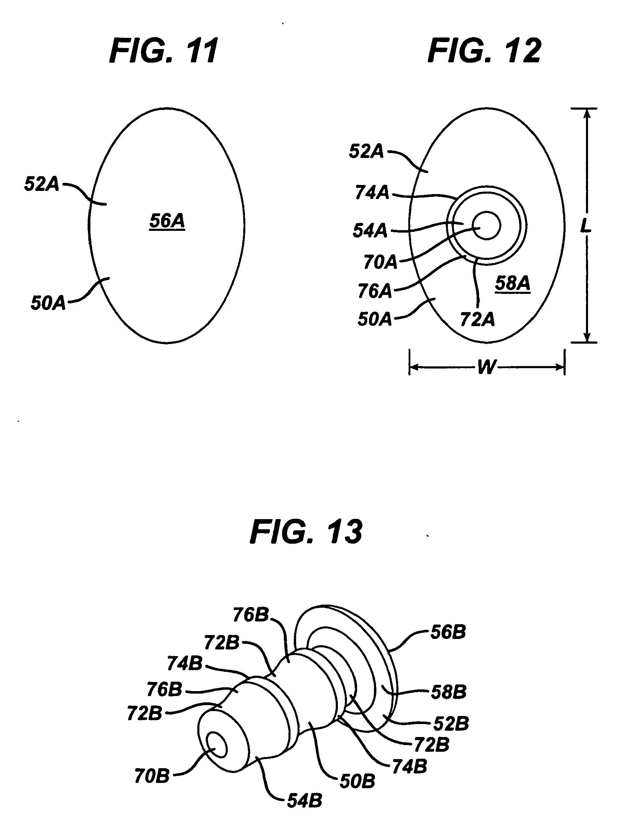

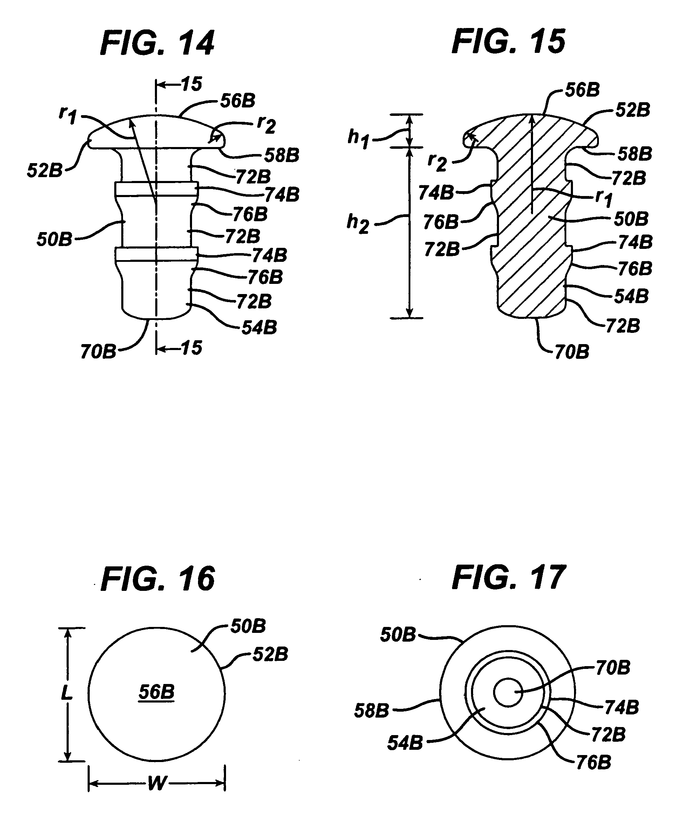

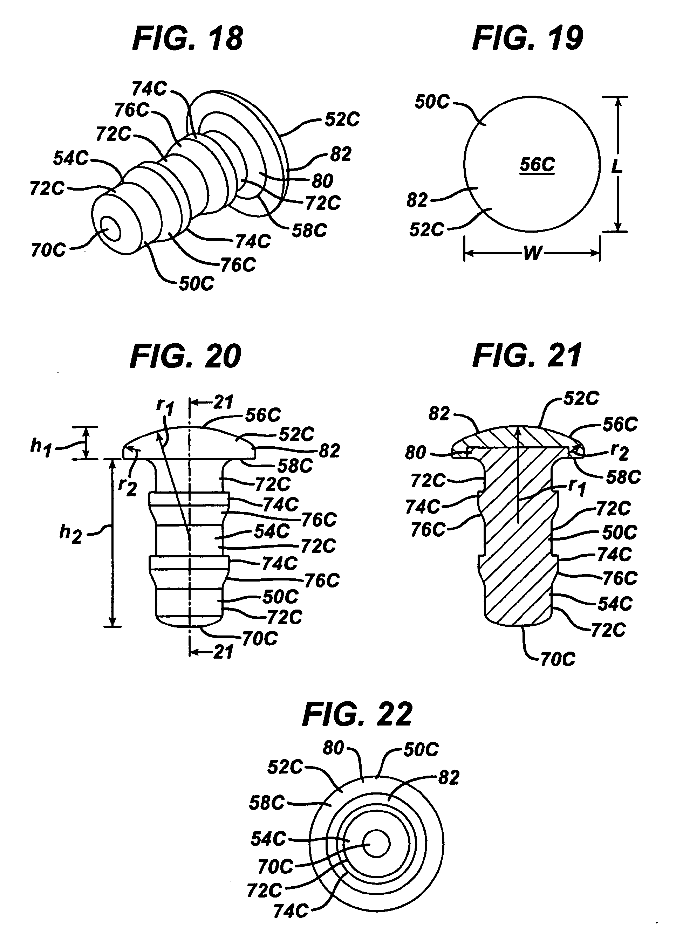

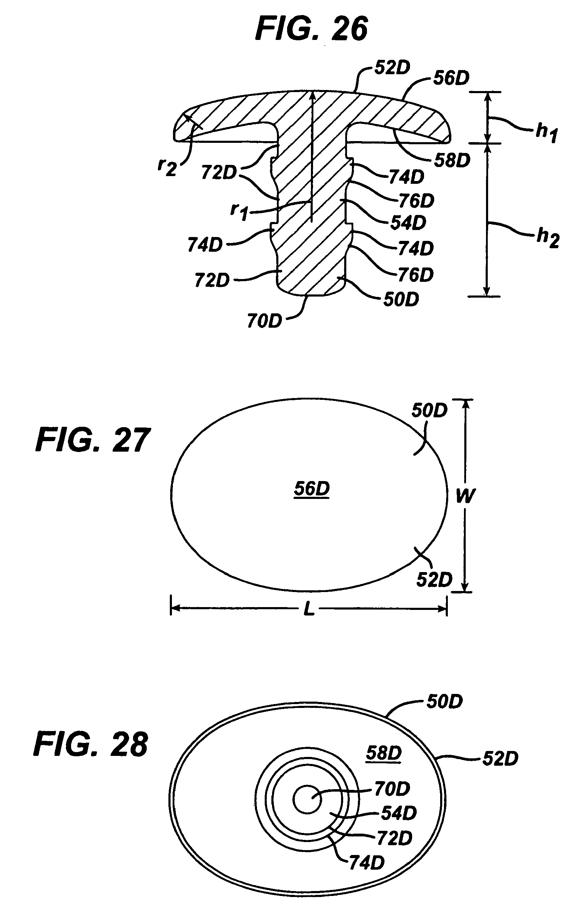

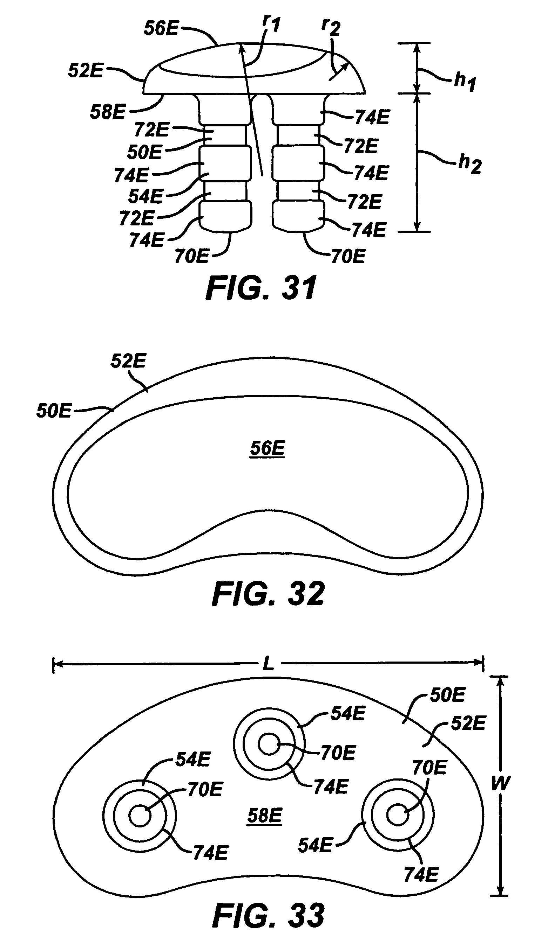

The head 52A, 52B, 52C, 52D, 52E of each of the illustrated anti-abrasion stud 50A, 50B, 50C, 50D, 50E is sized and shaped to fit between a portion of the trochlear component 12 and a portion of one uni-condylar femoral implant component 14 without contacting either the trochlear component or the uni-condylar femoral implant component when all of the components are implanted on the distal femur, as illustrated in FIGS. 5 and 6. As can also be seen from FIGS. 5-33, the head 52A, 52B, 52C, 52D, 52E of each anti-abrasion stud 50A, 50B, 50C, 50D, 50E has a shape that is different from the shape of the bearing surfaces of the trochlear implant component 12 and the uni-condylar femoral implant component 14. Two of the illustrated anti-abrasion studs 50A, 50D have heads that are elliptical in top plan view (see FIGS. 11 and 27); two of the illustrated anti-abrasion studs 50B, 50C have heads that are circular in top plan view (see FIGS. 16 and 19); and one of the illustrated anti-abrasion studs 50E has a head that is kidney-shaped in top plan view (see FIG. 32).

It should be appreciated that the three illustrated shapes for the heads of the anti-abrasion studs are provided as examples only. Alternative shapes may be used and are within the scope of the invention. For example, for anti-abrasion studs that are intended for use to extend the patellar tracking surface further toward the intercondylar notch, the heads of the anti-abrasions studs can have an edge that is shaped to complement the shape of a portion of the edge of the trochlear implant component.

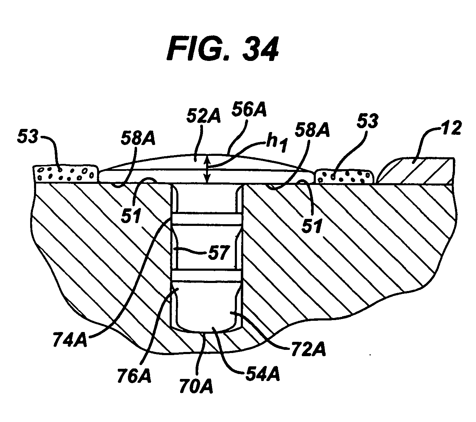

The head 52A, 52B, 52C, 52D, 52E of each of the illustrated anti-abrasion studs 50A, 50B, 50C, 50D, 50E has a height between the lowest portion of the bone-facing surface 58A, 58B, 58C, 58D, 58E and the highest point on the bearing surface 56A, 56B, 56C, 56D, 56E. These heights are indicated at "h₁" in FIGS. 8, 10, 15, 20, 25, 26 and 31. Generally, head heights h, are at least about 2 mm. The head heights h, are not more than about 6 mm. Such head heights should be adequate to raise most of the bearing surface 56 of the head 52 above the exterior surface of the articular cartilage on the bone; in other words, the head heights are generally greater than the thickness of the articular cartilage where the anti-abrasion stud is implanted. FIG. 34 illustrates the lowermost point of the bone-facing surface 54A of one of the anti-abrasion studs 50A positioned against the bone surface 51, with a substantial part of the bearing surface 56A of the head 52A above the top level of the articular cartilage 53 surrounding the anti-abrasion stud 50A. A portion of another implant component, such as trochlear component 12, is shown in cross-section in FIG. 34. Examples of numerical values for h₁ for the illustrated embodiments are provided in Table 1, below.

The head 52A, 52B, 52C, 52D, 52E of each of the illustrated anti-abrasion studs 50A, 50B, 50C, 50D and 50E has a maximum length and width. These lengths and widths are indicated at "L" and "w" in FIGS. 12, 16, 19, 27 and 33. Examples of numerical values for 1 and w for the illustrated embodiments are provided in Table 1, below. Examples of numerical values for the perimeters of the illustrated heads are also provided in Table 1 below.

All of the bearing surfaces 56A, 56B, 56C, 56D, 56E of the illustrated anti-abrasion studs 50A, 50B, 50C, 50D, 50E are contoured and substantially smooth, to provide a low friction path for the patellar component during flexion and extension. The illustrated bearing surfaces are convex. The radii of curvature for the bearing surfaces are indicated at "r₁" in FIGS. 8, 15, 21, 24, 26 and 30. Examples of numerical values for r₁ for the illustrated embodiments are provided in Table 1, below. Examples of surface areas for the bearing surfaces of the illustrated heads are also provided in Table 1 below.

It should be appreciated that the profiles of the bearing surfaces 56A, 56B, 56C, 56D, 56E of the illustrated embodiments are provided as examples only. Various profiles for the bearing surfaces could be used; the most appropriate profile for a bearing surface may relate to the shape of the bearing surface of the implant that the anti-abrasion stud is augmenting or extending. A particular profile or groups of profiles for the bearing surfaces of the anti-abrasion studs can be selected to best augment a wide variety of main implant shapes and sizes. For example, it may be desirable to include a concave portion to form a track. Accordingly, the present invention is not limited to any particular profile for the bearing surfaces of the anti-abrasion studs unless expressly called for in the claims.

The head 52A, 52B, 52C, 52D, 52E of each of the illustrated anti-abrasion studs 50A, 50B, 50C, 50D, 50E has a curved edge 60A, 60B, 60C, 60D, 60E around the perimeter of the bearing surface. The curved edges 60A, 60B, 60C, 60D, 60E extend toward the bone-facing surfaces 58A, 58B, 58C, 58D, 58E. In the illustrated embodiments the curved edges have radii of curvature of at least about 0.5 mm. The radii of curvature of the curved edges are not more than about 5mm. These radii are indicated at "r₂" in FIGS. 8, 10, 14, 15, 20, 21, 24, 26 and 31. Examples of numerical values for r₂ for the illustrated embodiments are provided in Table 1, below.

**Table 1**

| Stud ref | Dimension (mm) | | | | | | | Surface area (mm²) |
|---|---|---|---|---|---|---|---|---|
| | L | w | h₁ | h₂ | r₁ | r₂ | Perimeter | |
| 50A | 18 | 12 | 2.34 | 12.66 | 30 | 0.5 | 142.44 | 183.66 |
| 50B | 10 | 10 | 2.34 | 12.66 | 10 | 1 | 87.27 | 99.00 |
| 50C | 10 | 10 | 2.34 | 12.66 | 10 | 1 | 87.27 | 99.00 |
| 50D | 20 | 14 | 3.75 | 11.26 | 30 | 2 | 165.85 | 288.32 |
| 50E | 41 | 22 | 5.00 | 13.50 | 30 | 5 | 365.10 | 857.98 |

The fixation posts 54A, 54B, 54C, 54D, 54E of each of the illustrated embodiments of anti-abrasion studs 50A, 50B, 50C, 50D, 50E are provided for affixation of the studs to the patient's bone. The illustrated fixation posts are intended to be placed in a prepared bore in the patient's bone, such as the substantially cylindrical bore shown at 57 in FIG. 34, and include raised surface features to aid in affixation of the posts to the walls of the bore 57 in the bone.

Each of the illustrated fixation posts 54A, 54B, 54C, 54D, 54E has a flat, circular end 70A, 70B, 70C, 70D, 70E opposite the head 52A, 52B, 52C, 52D, 52E. The illustrated fixation posts include a plurality of spaced cylindrical portions 72A, 72B, 72C, 72D, 72E having a first diameter and spaced raised cylindrical portions 74A, 74B, 74C, 74D, 74E having a second larger diameter. The cylindrical portions 72A, 72B, 72C, 72D, 72E and raised cylindrical portions 74A, 74B, 74C, 74D, 74E are concentric about the longitudinal axes 75A, 75B, 75C, 75D, 75E of the fixation posts. In the anti-abrasion studs 50A, 50B, 50C, 50D illustrated in FIGS. 7-23, the fixation posts further include conical bevelled portions 76A, 76B, 76C, 76D connecting the raised cylindrical portions 74A, 74B, 74C, and 74D to the cylindrical portions 72A, 72B, 72C, and 72D. The conical bevelled portions 76A, 76B, 76C, 76D are concentric about the longitudinal axes 75A, 75B, 75C, 75D of the fixation posts and taper toward the flat circular ends 70A, 70B, 70C, and 70D of the fixation posts.

The number of fixation posts and the positions of the fixation posts relative to the heads may vary depending on the size and shape of the head. For example, in the first four illustrated anti-abrasion studs 50A, 50B, 50C, 50D, the longitudinal axes of the fixation posts 54A, 54B, 54C, 54D are aligned with the centres of the heads 52A, 52B, 52C, 52D. In the last illustrated anti-abrasion stud 50E, there are three spaced fixation posts 54E positioned to support the head 52E.

Examples of dimensions for the fixation posts 54A, 54B, 54C, 54D, 54E and their surface features 70, 72, 74 are provided in Table 2.

**Table 2**

| Stud ref | Diameter (mm) | | |
|---|---|---|---|
| | Circular end 70 | Smaller diameter cylindrical portion 72 | Larger diameter cylindrical portion 74 |
| 50A | 2 | 5 | 6 |
| 50B | 2 | 5 | 6 |
| 50C | 2 | 5 | 6 |
| 50D | 2 | 5 | 6 |
| 50E | 2 | 4 | 5 |

It should be understood that the surface features 70, 72, 74 described above are provided as examples only. Other surface features to aid in fixation of the anti-abrasion studs in the bone could be used in addition to or in place of the surface features illustrated and described above. For example, longitudinal surface features could be employed; grooves, ridges or fins could also be used to enhance fixation and retard rotation of the anti-abrasion studs.

It should also be understood that all of the dimensions, areas and radii disclosed herein (including all those set out in Tables 1 and 2) are provided as examples only.

In four of the illustrated anti-abrasion studs 50A, 50B, 50D, 50E, the entire head 52A, 52B, 52D and 52E and fixation post 54A, 54B, 54D, 54E are integrally-formed. However, the anti-abrasion studs could be made as multi-piece implants that can be assembled in the operating room. The anti-abrasion stud 50C of FIGS. 18-22 is an example of a two-piece anti-abrasion stud, wherein the fixation post 54C includes a flange 80 to which an independent bearing 82 is affixed. Together, the flange 80 and bearing 82 form the head 52C of the stud 50C. The bearing 82 can be affixed to the flange 80 in any standard manner, such as through an interference fir or frictional lock. With such a two-piece stud, a surgical kit could be modular, including a plurality of bearings 82 of different sizes and shapes from which the surgeon may select the most appropriate size and shape for the particular patient.

The anti-abrasion studs 50A, 50B, 50C, 50D, 50E of the present invention may be made of any standard bio-compatible material, although it is preferred that the material be one that is not biodegradable and not bioresorbable. Common metal alloys, such as standard medical implant grade cobalt-chrome alloys and titanium alloys, may be used for the entire implant. In the case of a two-piece anti-abrasion stud 50C of FIGS. 18-22, the fixation post 54C and flange 80 may be made of such a standard material, and the bearing 82 may be made of a different material, such as a ceramic or polymer (for example, ultra-high molecular weight polyethylene), if desired. The entire anti-abrasion stud could also be made of such a ceramic or polymer.

If all or part of the anti-abrasion stud is made of a metal alloy, it may be desirable for the surfaces that will contact bone to be treated to be conducive to bone ingrowth. For example, standard industry can be employed to make the bone-contacting surfaces porous. Coatings may also be employed to induce bone ingrowth into the appropriate portions of the stud or to deliver drugs to the site.

The bearing surfaces 56A, 56B, 56C, 56D, 56E of the anti-abrasion studs preferably provide a low-friction surface for the patellar bearing to move across during the flexion and extension. If the heads 52A, 52B, 52C, 52D, 52E are made of metal, the bearing surfaces may be highly polished to maximize smooth movement of the patellar bearing across the stud bearing surface.

The anti-abrasion studs of the present invention may be provided in the form of implant system, sets or kits. For example, a knee implant system, set or kit could include a set of trochlear components, patellar components and anti-abrasion studs. The system, set or kit could also include uni-condylar femoral implant components and uni-condylar tibial implant components. All of the implant components could be provided in a variety of sizes to accommodate a wide range of patient anatomies. The anti-abrasion studs included in the system, set or kit could include a variety of sizes of a single head shape or a variety of head shapes, profiles and sizes.

To use the anti-abrasion studs 50A, 50B, 50C, 50D, 50E and implant systems of the present invention, the orthopaedic surgeon would prepare the patient's bones in the most appropriate fashion for implantation of the first or major implant components. For example, for a patellofemoral joint arthroplasty, the trochlea of the distal femur would be resected or otherwise shaped or prepared to receive the trochlear implant component and the patella would be resected or otherwise shaped or prepared to receive the patellar implant component (if a patellar implant component is to be used). The trochlear component would then be implanted in a standard manner, as would the patellar implant component, if used. For a tibiofemoral joint arthroplasty, one or both of the femoral condyles would be resected or otherwise shaped or prepared to receive an appropriate uni-condylar femoral implant component and the corresponding side of the tibial plateau would be resected or otherwise shaped or prepared to receive an appropriate tibial implant component (or assembly of components). The femoral uni-condylar implant component or components and the uni-condylar tibial component or components would then be implanted in a standard manner.

If the surgeon determines at the time of the original surgery that the patient would benefit from providing an enhanced or augmented patellar track extending further toward the intercondylar notch, or that the transition between the bearing surfaces of the trochlear component and the uni-condylar femoral component or components is uneven or overly extended, the surgeon may chose to use one of the anti-abrasion studs of the system to extend the bearing surfaces of the other implant components.

The orthopaedic surgeon may select the most appropriate size and shape of anti-abrasion stud to extend the bearing surface or surfaces. Preferably, the head of the anti-abrasion stud is sized and shaped so that it will not contact any part of the trochlear implant component or uni-condylar femoral implant component. A drill or reamer is then used to prepare a bore in the bone; preferably, the outer diameter of the drill or reamer is slightly less than the outer diameter of the fixation feature (such as larger diameter portion 74) of the fixation post. The fixation post is then introduced into the bore and pushed into the bore until the lowermost part of the head (such as the bone-facing portion) contacts the surface of the bone. At least a substantial part of the bearing surface of the head will be above the level of the articular cartilage. This procedure may be repeated with additional anti-abrasion studs as deemed necessary by the surgeon.

If the orthopaedic surgeon initially elects to avoid using the anti-abrasion studs, the studs may be implanted in a separate procedure on a later date. For example, if the patient has received a trochlear implant or a uni-condylar femoral implant and complains of pain or of a patellar component catching or making a noise during flexion or extension, the surgeon may opt to implant an anti-abrasion stud at that time. Due to the small size of the anti-abrasion studs, this subsequent procedure can be a minimally invasive one.

Thus, the system of the present invention provides the surgeon with the opportunity to enhance and extend the bearing surfaces of standard uni-compartmental implant components to fit the needs of individual patients. The anti-abrasion stud will provide an additional bearing surface that substantially bridges a portion of the gap between the other implant components to provide an augmented bearing surface and covers and protects the native articular cartilage from abrasion.

## Claims

1. An orthopaedic implant system for use in treating an orthopaedic disorder of a patello-femoral joint between the femur and the tibia and a tibio-femoral joint between the femur and the patella, the femur including native articular cartilage, the implant system comprising:
a trochlear implant component sized and shaped to replace a portion of the femur without covering the distal surfaces of the medial and lateral condyles, the trochlear component having a bearing surface and a bone-facing surface;
a uni-condylar implant component sized and shaped to replace a portion of one of the condyles of the distal femur, the uni-condylar implant component having a bearing surface and a bone-facing surface; and
a patellar implant component sized and shaped to replace a portion of the patella, the patellar implant component having a bearing surface and a bone-facing surface;
**characterized in that** the orthopaedic implant system further comprises
an implantable stud including a head having a bearing surface and a bone-facing surface, the implantable stud further including a fixation post extending outward from the bone-facing surface of the head, the head of the stud being sized and shaped to fit between a portion of the trochlear component and a portion of the uni-condylar implant component without contacting either the trochlear component or the uni-condylar implant component when all three components are implanted on the distal femur;
wherein the bearing surface of the head has a different shape than the bearing surfaces of the trochlear implant component and the uni-condylar implant component; and
wherein the bearing surface of the head is sized and shaped to limit contact between the patellar implant component and native tissue during flexion and extension of the knee joint.

2. The orthopaedic implant system of claim 1 wherein the area of the bearing surface of the head of the stud is less than 900 mm² and the bearing surface of the head of the implantable stud is convex and substantially smooth.

3. The orthopaedic implant system of claim 2 wherein the area of the bearing surface of the head of the stud is not more than about 300 mm², preferably not more than about 200 mm², especially not more than about 100 mm².

4. The orthopaedic implant system of claim 2 wherein the area of the bearing surface of the head of the stud is at least about 90 mm².

5. The orthopaedic implant system of claim 2 wherein the bearing surface of the head of the implantable stud has a radius of curvature of at least about 10 mm.

6. The orthopaedic implant system of claim 2 wherein the bearing surface of the head of the implantable stud has a radius of curvature of not more than about 30 mm.

7. The orthopaedic implant system of claim 2 wherein the fixation post has a plurality of cylindrical portions having a first diameter and raised cylindrical portions having a second larger diameter.

8. The orthopaedic implant system of claim 2 wherein the shape of the head when viewed in top plan view is circular or elliptical or kidney-shaped.

9. The orthopaedic implant system of claim 2 further comprising a uni-condylar implant component sized and shaped to replace a portion of the distal surface of one of the condyles of the distal femur.

10. The orthopaedic implant system of claim 2 wherein the fixation post of the implantable stud is porous.

## Patentansprüche

1. Orthopädisches Implantatsystem zur Verwendung beim Behandeln einer orthopädischen Erkrankung einer patellofemoralen Verbindungsstelle zwischen dem Oberschenkelknochen und dem Schienbein und einer tibiofemoralen Verbindungsstelle zwischen dem Oberschenkelknochen und der Kniescheibe, wobei der Oberschenkelknochen nativen Gelenkknorpel umfasst, wobei das Implantatsystem aufweist:
eine Trochlea-Implantatkomponente, die so bemessen und geformt ist, dass sie einen Teil des Oberschenkelknochens ersetzt, ohne die distalen Flächen der medialen und lateralen Kondylen abzudecken, wobei die Trochlea-Komponente eine Lagerfläche und eine zum Knochen weisende Fläche hat;
eine Unikondylen-Implantatkomponente, die so bemessen und geformt ist, dass sie einen Teil einer der Kondylen des distalen Oberschenkelknochens ersetzt, wobei die Unikondylen-Implantatkomponente eine Lagefläche und eine zum Knochen weisende Fläche hat; und
eine Kniescheiben-Implantatkomponente, die so bemessen und geformt ist, dass sie einen Teil der Kniescheibe ersetzt, wobei die Kniescheiben-Implantatkomponente eine Lagerfläche und eine zum Knochen weisende Fläche hat;
**dadurch gekennzeichnet, dass** das orthopädische Implantatsystem weiter aufweist:
einen implantierbaren Zapfen, der einen Kopf umfasst, welcher eine Lagerfläche und eine zum Knochen weisende Fläche hat, wobei der implantierbare Zapfen weiter einen Fixierstift umfasst, der sich von der zum Knochen weisenden Fläche des Kopfes nach außen erstreckt, wobei der Kopf des Zapfens so bemessen und geformt ist, dass er zwischen einen Teil der Trochlea-Komponente und einen Teil der Unikondylen-Implantatkomponente passt, ohne die Trochlea-Komponente oder die Unikondylen-Implantatkomponente zu berühren, wenn alle drei Komponenten auf dem distalen Oberschenkelknochen implantiert sind;
wobei die Lagerfläche des Kopfes eine andere Form hat als die Lagerflächen der Trochlea-Implantatkomponente und der Unikondylen-Implantatkomponente; und
wobei die Lagerfläche des Kopfes so bemessen und geformt ist, dass sie den Kontakt zwischen der Kniescheiben-Implantatkomponente und dem nativen Gewebe während des Beugens und Streckens des Kniegelenks begrenzt.

2. Orthopädisches Implantatsystem nach Anspruch 1, bei dem die Fläche der Lagerfläche des Kopfes des Zapfens weniger als 900 mm² beträgt und die Lagerfläche des Kopfes des implantierbaren Zapfens konvex und im Wesentlichen glatt ist.

3. Orthopädisches Implantatsystem nach Anspruch 2, bei dem die Fläche der Lagerfläche des Kopfes des Zapfens nicht mehr als ungefähr 300 mm² beträgt, bevorzugt nicht mehr als ungefähr 200 mm², insbesondere nicht mehr als ungefähr 100 mm².

4. Orthopädisches Implantatsystem nach Anspruch 2,bei dem die Fläche der Lagerfläche des Kopfes des Zapfens wenigstens ungefähr 90 mm² beträgt.

5. Orthopädisches Implantatsystem nach Anspruch 2, bei dem die Lagerfläche des Kopfes des implantierbaren Zapfens einen Krümmungsradius von wenigstens ungefähr 10 mm hat.

6. Orthopädisches Implantatsystem nach Anspruch 2, bei dem die Lagerfläche des Kopfes des implantierbaren Zapfens einen Krümmungsradius von nicht mehr als ungefähr 30 mm hat.

7. Orthopädisches Implantatsystem nach Anspruch 2, bei dem der Fixierstift eine Vielzahl zylindrischer Abschnitte mit einem ersten Durchmesser und angehobene zylindrische Abschnitte mit einem zweiten größeren Durchmesser hat.

8. Orthopädisches Implantatsystem nach Anspruch 2, bei dem die Form des Kopfes, wenn in Draufsicht betrachtet wird, kreisförmig oder elliptisch oder nierenförmig ist.

9. Orthopädisches Implantatsystem nach Anspruch 2, weiter mit einer Unikondylen-Implantatkomponente, die so bemessen und geformt ist, dass sie einen Teil der distalen Fläche einer der Kondylen des distalen Oberschenkelknochens ersetzt.

10. Orthopädisches Implantatsystem nach Anspruch 2, bei dem der Fixierstift des implantierbaren Zapfens porös ist.

## Revendications

1. Système d'implant orthopédique destiné à être utilisé pour traiter un problème orthopédique d'une articulation fémoro-rotulienne entre le fémur et le tibia et une articulation fémoro-tibiale entre le fémur et la rotule, le fémur comprenant du cartilage articulaire natif, le système d'implant comprenant :
■ un composant d'implant trochléaire dimensionnée et formé pour remplacer une partie du fémur sans recouvrir les surfaces distales des condyles interne et externe, le composant trochléaire ayant une surface d'appui et une surface orientée vers l'os ;
■ un composant d'implant unicondylien dimensionné et formé pour remplacer une partie de l'un des condyles du fémur distal, le composant d'implant unicondylien ayant une surface d'appui et une surface orientée vers l'os.; et
■ un composant d'implant rotulien dimensionné et formé pour remplacer une partie de la rotule, le composant d'implant rotulien ayant une surface d'appui et une surface orientée vers l'os ;
**caractérisé en ce que** le système d'implant orthopédique comprend en outre :
■ un goujon implantable comprenant une tête ayant une surface d'appui et une surface orientée vers l'os, le goujon implantable comprenant en outre un montant de fixation s'étendant vers l'extérieur à partir de la surface orientée vers l'os, de la tête, la tête du goujon étant dimensionnée et formée pour s'adapter entre une partie du composant trochléaire et une partie du composant d'implant unicondylien sans entrer en contact avec le composant trochléaire ni avec le composant d'implant unicondylien lorsque tous les trois composants sont implantés sur le fémur distal ;
■ dans lequel la surface d'appui de la tête a une forme différente des surfaces d'appui du composant d'implant trochléaire et du composant d'implant unicondylien ; et
■ dans lequel la surface d'appui de la tête est dimensionnée et formée pour limiter le contact entre le composant d'implant rotulien et le tissu natif pendant la flexion et l'extension de l'articulation du genou.

2. Système d'implant orthopédique selon la revendication 1, dans lequel l'aire de la surface d'appui de la tête de goujon est inférieure à 900 mm², et la surface d'appui de la tête du goujon implantable est convexe et sensiblement lisse.

3. Système d'implant orthopédique selon la revendication 2, dans lequel la surface de la surface d'appui de la tête du goujon n'est pas supérieure à environ 300 mm², de préférence non supérieure à environ 200 mm², en particulier non supérieure à 100 mm².

4. Système d'implant orthopédique selon la revendication 2, dans lequel l'aire de la surface d'appui de la tête du goujon est d'au moins environ 90 mm².

5. Système d'implant orthopédique selon la revendication 2, dans lequel la surface d'appui de la tête de goujon implantable a un rayon de courbure d'au moins environ 10 mm.

6. Système d'implant orthopédique selon la revendication 2, dans lequel la surface d'appui de la tête du goujon implantable a un rayon de courbure non supérieur à environ 30 mm.

7. Système d'implant orthopédique selon la revendication 2, dans lequel le montant de fixation a une pluralité de parties cylindriques ayant un premier diamètre et des parties cylindriques relevées ayant un second diamètre plus important.

8. Système d'implant orthopédique selon la revendication 2, dans lequel la forme de la tête lorsqu'elle est observée sur une vue en plan de dessus, est circulaire ou elliptique ou en forme de rein.

9. Système d'implant orthopédique selon la revendication 2, comprenant en outre un composant d'implant unicondylien dimensionné et formé pour remplacer une partie de la surface distale de l'un des condyles du fémur distal.

10. Système d'implant orthopédique selon la revendication 2, dans lequel le montant de fixation du goujon implantable est poreux.
